# EUROPÄISCHE PATENTSCHRIFT

(11) **EP 4 367 106 B1**
(45) Veröffentlichungstag und Bekanntmachung des Hinweises auf die Patenterteilung: **11.11.2026**
(21) Anmeldenummer: 22743805.8
(22) Anmeldetag: 04.07.2022
(51) Int. Cl.: C07D 271/113, C07C 317/44, A01N 37/40, A01P 3/00

(54) **CHIRALE 3-SULFINYLBENZOESÄUREN**
CHIRAL 3-SULFINYL BENZOIC ACIDS
ACIDES 3-SULFINYLBENZOÏQUES CHIRAUX

(30) Priorität: 08.07.2021 EP 21184514
(43) Veröffentlichungstag der Anmeldung: 15.05.2024
(73) Patentinhaber: Bayer Aktiengesellschaft, 51373 Leverkusen (DE)
(72) Erfinder: PAZENOK, Sergii, 51373 Leverkusen (DE); HEILMANN, Eike Kevin, 51373 Leverkusen (DE); SCHIRMER, Heiko, 51373 Leverkusen (DE); SCHIFFER, Klaus-Ulrich, 51373 Leverkusen (DE); LOVIS, Kai, 51373 Leverkusen (DE); KQIKU, Laura, 51373 Leverkusen (DE)
(74) Vertreter: BIP Patents
(86) Internationale Anmeldenummer: PCT/EP2022/068443
(87) Internationale Veröffentlichungsnummer: WO 2023/280773

(56) Entgegenhaltungen:
- WO-A1-2016/001073
- WO-A1-2018/202535
- US-A1- 2012 165 540

## Beschreibung

Die Erfindung betrifft chirale 3-Sulfinylbenzoesäuren, deren Verwendung sowie ein Verfahren zur Herstellung chiraler N-(1,2,5-Oxadiazol-3-yl)-, N-(1,3,4-Oxadiazol-2-yl)-, N-(Tetrazol-5-yl)- und N-(Triazol-5-yl)phenylcarbonsäureamide.

WO 2018/202535 A1, WO 2016/001073 A1 und US 2012/0165540 A1 offenbaren jeweils bestimmte racemische 3-Sulfinylbenzoesäuren bzw. deren Verwendung zur Herstellung bestimmter Herbizide.

Aus WO 2021/078174 A1 sind herbizid wirksame chirale N-(1,2,5-Oxadiazol-3-yl)-, N-(1,3,4-Oxadiazol-2-yl)-, N-(Tetrazol-5-yl)- und N-(Triazol-5-yl)phenylcarbonsäureamide bekannt. Aus EP 21162218 sind ebenfalls herbizid wirksame chirale N-(1,3,4-Oxadiazol-2-yl)phenylcarbonsäureamide bekannt. Die dort beschriebenen herbizid wirksamen chiralen Verbindungen tragen in 3-Stellung des Phenylrings eine chirale Sulfinylgruppe. Diese Verbindungen werden aufwändig durch Enantiomerentrennung der N-(1,2,5-Oxadiazol-3-yl)-, N-(1,3,4-Oxadiazol-2-yl)-, N-(Tetrazol-5-yl)- und N-(Triazol-5-yl)phenylcarbonsäureamide hergestellt.

Aufgabe der vorliegenden Erfindung war es die aus dem Stand der Technik bekannten Nachteile zu überwinden.

Ein Gegenstand vorliegender Erfindung sind chirale 3-Sulfinylbenzoesäuren der in Formel (I-S) angegebenen absoluten Konfiguration 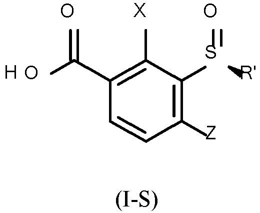 worin die Substituenten folgende Bedeutungen haben:
- X: bedeutet Cl oder Methyl,
- R': bedeutet Methyl oder c-Pr,
- Z: bedeutet CF₃ oder CHF₂.

Erfindungsgemäße Verbindungen sind solche Verbindungen der allgemeinen Formel (I-S), welche nach den Cahn-Ingold-Prelog Regeln in der S-Konfiguration vorliegen sofern R' eine geringere Priorität als der Phenylring aufweist. Dieses gilt zum Beispiel für Verbindungen der allgemeinen Formel (I), bei denen R' Methyl oder Cyclopropyl ist. Weitere erfindungsgemäße Verbindungen sind solche Verbindungen der allgemeinen Formel (I), welche nach den Cahn-Ingold-Prelog Regeln in der R-Konfiguration vorliegen sofern R' eine höhere Priorität als der Phenylring aufweist. Dieses gilt zum Beispiel für Verbindungen der allgemeinen Formel (I), bei denen R' Methoxymethyl ist.

In Formel (I-S) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Durch Halogen substitiertes Alkyl bedeutet geradkettige oder verzweigte Alkylgruppen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Halogen steht für Fluor, Chlor, Brom oder Iod.

Ist eine Gruppe mehrfach durch Reste substituiert, so ist darunter zu verstehen, daß diese Gruppe durch ein oder mehrere gleiche oder verschiedene der genannten Reste substituiert ist.

In allen nachfolgend genannten Formeln haben die Substituenten und Symbole, sofern nicht anders definiert, dieselbe Bedeutung wie unter Formeln (I-S) beschrieben. OMe bedeutet O-Methyl; SMe bedeutet S-Methyl; SEt bedeutet S-Ethyl; CH₂OMe bedeutet CH₂O-Methyl; i-Pr bedeutet iso-Propyl; c-Pr bedeutet cyclo-Propyl.

Erfindungsgemäße Verbindungen der allgemeinen Formel (I-S) können beispielsweise durch nachfolgend beschriebene Verfahren aus den jeweiligen racemischen Verbindungen (I-rac) hergestellt werden. Diese Verfahren sind ebenfalls ein Gegenstand vorliegender Erfindung.

Die racemischenn Verbindungen (I-rac) beziehungsweise deren Herstellung sind z.B. aus WO 2021/078174 A1 und WO 2012/126932 A1 grundsätzlich bekannt. Die racemischen Verbindungen (I-rac) werden mit einem enantiomerenreinen Amin der allgemeinen Formel (II) umgesetzt, wobei unter geeigneten Bedingungen nur eines der beiden möglichen diastereomeren Salze (III-dR) oder (III-dS) auskristallisiert und zur weiteren Aufarbeitung abgetrennt werden kann. Das andere diastereomere Salz kann aus der Mutterlauge isoliert werden.

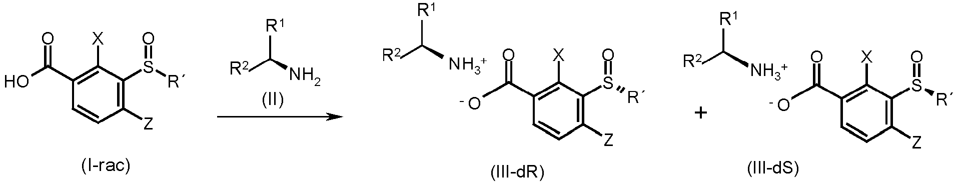

Die Kristallisation kann in verschiedenen geeigneten Lösungsmitteln oder Lösungsmittelgemischen stattfinden, wobei Methanol, Methanol / Wasser (1:1 bis 10:1), Ethanol / Wasser (1:1 bis 10:1), Isopropanol, bevorzugt werden IsoPropanol / Wasser (Bereich 1:1 bis 10:1), Aceton / Wasser (1:1 bis 20:1), Ethylacetat, THF, THF / Wasser (3:1 bis 20:1) oder Toluol. Die erhaltenen Salzkristalle werden unter Verwendung der bekannten Methoden durch Filtration von der Mutterlauge abgetrennt und mit dem verwendeten Lösungsmittel oder Lösungsmittelgemisch gewaschen und im Vakuum getrocknet. In einem weiteren Reaktionsschritt werden die isolierten diastereomeren Verbindungen der allgemeinen Formel (III-dR) oder (III-dS) dann mit Wasser bei einer Temperatur von 0 °C bis 20 °C gemischt, gegebenenfalls in Gegenwart von organischen Lösungsmitteln wie Methanol, Ethanol, Iso- Propanol, THF, Aceton usw. und mit einer starken Säure wie HCl oder H₂SO₄ versetzt, um einen pH-Wert von 1-2 zu erreichen. Die enantiomerenreinen Verbindungen der allgemeinen Formel (I-S) fallen aus und werden durch Filtration von der Mutterlauge abgetrennt, gewaschen und im Vakuum getrocknet.

Die Verbindungen der Formel (I-rac) und das Amin der Formel (II) werden üblicherweise in äquimolaren Mengen eingesetzt. Dieser Schritt wird normalerweise bei Raumtemperatur ausgeführt.

Als chirale Amine sind eine Vielzahl käuflich erhältlicher Amine der Formel (II) geeignet, z.B. solche, in denen
- R¹: für Methyl, Ethyl, n-Propyl, iso-Propyl, n-Butyl, iso-Butyl steht, und
- R²: für Hydroxymethyl, Phenyl, 4-Methylphenyl steht.

Beispielsweise sind folgende folgende Amine der Formel (II) gut geeignet:

(S)-(-)-α,4-Dimethylbenzylamin (CAS-No. 27298-98-2),

(R)-(-)-3-Methyl-2-phenylbutylamin (CAS-No. 67152-35-6),

(S)-(+)-2-Amino-3-methyl-1-butanol (CAS-No. 2026-48-4).

Bevorzugt ist (S)-(-)-α,4-Dimethylbenzylamin (CAS-Nr. 27298-98-2).

Die erfindungsgemäßen 3-Sulfinylbenzoesäuren der Formel (I-S) werden bei dem vorstehend genannten Verfahren in der Regel mit einem Enantiomerenüberschuss (ee) von mindestens 94%, oftmals auch mindestens 99% gewonnen.

3-Sulfinylbenzoesäuren der Formel (I-S) mit einem Enantiomerenüberschuss (ee) von mindestens 94% sind bevorzugt. 3-Sulfinylbenzoesäuren der Formel (I-S) mit einem Enantiomerenüberschuss (ee) von mindestens 99% sind besonders bevorzugt.

Erfindungsgemäße 3-Sulfinylbenzoesäuren der allgemeinen Formeln (I-S) eignen sich besonders gut zur Herstellung von herbizid wirksamen Verbindungen wie sie in EP 21162218 beschrieben sind.

Ein weiterer Gegenstand vorliegender Erfindung ist somit ein Verfahren zur Herstellung von N-(1,3,4-Oxadiazol-2-yl)phenylcarbonsäureamiden mit der in Formel (I*) angegebenen absoluten Konfiguration durch Umsetzung von 2-Amino-1,3,4-oxadiazolen der allgemeinen Formel (III) mit erfindungsgemäßen 3-Sulfinylbenzoesäuren der allgemeinen Formel (I-S), 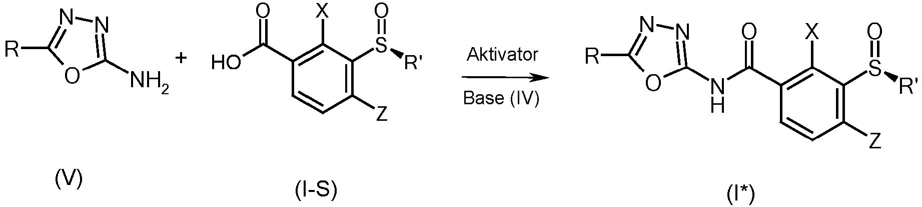 dadurch gekennzeichnet, dass es
a) in Gegenwart eines aktivierendes Reagenzes (Aktivator) aus der Gruppe bestehend aus Thionylchlorid, Phosgen, Diphosgen, Mesylchlorid, Tosylchlorid, POCl₃, PCl₅, Oxalylchlorid und C₁-C₈-alkyl-OC(O)Cl, und
b) in Gegenwart einer Base der allgemeinen Formel (IV) 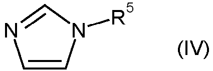 durchgeführt wird,
   und
c) worin die Substituenten wie nachfolgend definiert sind:
   - R: bedeutet Wasserstoff oder Methyl,
   - X: bedeutet Cl oder Methyl,
   - R': bedeutet Methyl oder c-Pr,
   - Z: bedeutet CF₃ oder CHF₂,
   - R⁵: bedeutet C₁-C₁₂-Alkyl oder Phenyl.

In dem zuvor beschriebenen Verfahren zur Herstellung von Verbindungen der Formel (I*) aus Verbindungen der Formel (V) und (I-S) werden die Verbindungen der Formeln (V) und (I-S) üblicherweise in einem Mol-Verhältnis von 0,8 bis 1,5 eingesetzt. Vorzugsweise wird die Verbindung der Formel (V) mit einem Überschuß von 10% zur Verbindung der Formel (I-S) eingesetzt.

Der Aktivator und die Verbindungen der Formel (I-S) werden üblicherweise in einem Mol-Verhältnis von 0,5 bis 3, bevorzugt von 1 bis 2, besonders bevorzugt von 1,2 bis 1,9, eingesetzt.

Vorzugsweise wird als Aktivator Thionylchlorid, Phosgene oder Diphosgen, besonders bevorzugt Thionylchlorid eingesetzt.

Die Base der Formel (IV) und die Verbindungen der Formel (I-S) werden üblicherweise in einem Mol-Verhältnis von 0,5 bis 10, bevorzugt von 1 bis 3, besonders bevorzugt von 1 bis 2,5, eingesetzt.

Die beiden zuvor genannten erfindungsgemäßen Verfahren zur Herstellung der Verbindungen der Formeln (I*) werden in der Regel in einem Lösungsmittel durchgeführt. Geeignete Lösungsmittel sind inerte organische Lösungsmittel, vorzugsweise aliphatische, alicyclische oder aromatische Kohlenwasserstoffe, wie Petrolether, Hexan, Heptan, Cyclohexan, Methylcyclohexan, Benzol, Toluol, Xylol und Decalin; halogenierte Kohlenwasserstoffe, wie Chlorobenzol, Dichlorobenzol, Dichlormethan, Chloroform, Tetrachloromethan, Dichlorethan und Trichlorethan; Ester wie Ethylacetat und Isopropylacetat; Ether wie Diethylether, Diisopropylether, Methyl-t-butylether, Methyl-t-amylether, Dioxan, Tetrahydrofuran, 1,2-Dimethoxyethan, 1,2-Diethoxyethan und Anisol; Ketone wie Aceton, Butanon, Methyl-isobutylketon und Cyclohexanon; Nitrile wie Acetonitril, Propionitril, n- oder i-Butyronitril und Benzonitril; Amide wie N,N-Dimethylformamid, N,N-Dimethylacetamid, N-Methylformanilid, N-Methylpyrrolidon und Hexamethylphosphoramid; Pyridine wie 2-Methylpyridin, 3-Methylpyridin, 4-Methylpyridin, 2,3-Dimethylpyridin, 2-Methyl-5-ethylpyridin, 2,6-Dimethylpyridin, 2,4-Dimethylpyridin, 3,4-Dimethylpyridin und 2,4,6-Trimethylpyridin. Ebenso sind Gemische der vorstehend genannten Lösungsmittel geeignet.

Vorzugsweise wird als Lösungsmittel Tetrahydrofuran, Acetonitril, 3-Methylpyridin oder 2-Methyl-5-ethylpyridin verwendet. Besonders bevorzugt ist 3-Methylpyridin.

Üblicherweise werden diese Verfahren in einem Temperaturbereich von -5° bis 50°C, vorzugsweise 0° bis 25°C durchgeführt.

Diese Verfahren werden üblicherweise so durchgeführt, daß die Verbindungen der Formeln (III), (I-S) und (IV) in einem Lösungsmittel vorgelegt werden und der Aktivator unter Rühren langsam zugetropft, oder eingeleitet im Falle von Phosgen, wird. Das Fortschreiten der Reaktion kann durch HPLC-Kontrolle verfolgt werden. Die vollständige Umsetzung ist in der Regel nach 10 bis 20 Stunden erfolgt.

Nach vollständiger Umsetzung wird die Reaktionsmischung abgekühlt und das Produkt fällt in der Regel schon nahezu quantitativ aus. Alternativ kann das Reaktionsgemisch mit einem polaren Lösungsmittel, wie Wasser oder Alkoholen wie Isopropanol, verdünnt werden. Das Reaktionsprodukt der Formel (I*) fällt in hoher Reinheit an und kann, falls erforderlich, weiter gereinigt werden. Besonders vorteilhaft ist es, das Reaktionsgemisch bei einer Temperatur zwischen 20 und 35 °C innerhalb von 3 bis 6 Stunden mit Wasser zu versetzen. Dabei fällt das Produkt in einer schnell filtrierbaren Form an. Nach Behandeln der Mutterlauge mit Natronlauge kann destillativ die Base der Formel (IV) zu etwa 95% wiedergewonnen werden.

Die nachstehenden Beispiele erläutern die Erfindung.

### Beispiel 1: Herstellung von 2-Chlor-3-(S)-methylsulfinyl]-4-(trifluormethyl)benzoesäure

### Schritt 1: Herstellung von 2-Chlor-3 [(S,R)-methylsulfinyl]-4-(trifluoromethyl)benzoesäure

In einem gerührten 3 Liter Doppelmantelreaktor werden 1 L Eisessig vorgelegt und anschließend 0,2 kg 2-Chlor-3-methylsulfanyl-4-(trifluoromethyl)benzoesäure zugegeben. Die trübe Mischung wird auf 60°C erwärmt und bei dieser Temperatur werden dann innerhalb von 130 min eine 35%ige wässrige Wasserstoffperoxidlösung zugetropft und für 21 Stunden bei 70°C Innentemperatur gerührt. Das Gemisch wird auf 20°C gekühlt und 100 ml einer 39%igen Natriumhydrogensulfit-Lösung zugetropft. Die Mischung wird dann in einem Rotationsverdampfer bis auf ein Restvolumen von ca. 20% eingeengt.

Der Rückstand wird in 1 l Wasser aufgenommen und mit 120 ml einer 45%igen Natronlauge alkalisch gestellt (pH 13-14). Die wässrige Lösung wird dann mit Dichlormethan gewaschen und die abgetrennte wässrige Phase auf 5°C gekühlt sowie mit 280 ml einer 32%igen Salzsäure angesäuert. Das Produkt fällt als Öl aus und kristallisiert nach wenigen Minuten. Der Feststoff wird über eine Filternutsche kalt abfiltriert und mit Wasser gewaschen und getrocknet. Man erhält 194 g eines beigen Feststoffs.

| | |
|---|---|
| HPLC (H₃PO₄): | logP = 0.96; |
| Massenspektrometrie: | 287.0 (M+H)⁺, 328.1 (M+H+CH₃CN)⁺, 573.0 (2M+H)⁺; |
| ¹H-NMR [DMSO-D₆]: | 14.2 (br s, 1H), 7.96-8.00 (m, 2H), 3.14 (s, 3H). |

### Schritt 2: Herstellung von 2-Chlor-3 [(S,R)-methylsulfinyl]-4-(trifluoromethyl)benzoesäure 2-Chlor-3-[(S)-methylsulfinyl]-4-(trifluoromethyl) benzoesäure [(1S)-1-(p-tolyl)ethyl]ammonium

In einem inertisierten und gerührten Doppelmantelreaktor werden 1,06 kg racemisches 2-Chlor-3 [*S,R*)-methylsulfinyl]-4-(trifluoromethyl)benzoesäure in 20 l Aceton gelöst und auf 55°C temperiert. Bei leichtem Rückfluss werden 519,4g (S)-(-)-α,4-Dimethylbenzylamin innerhalb von vier Stunden zugetropft und die resultierende Suspension bei 52°C über Nacht nachgerührt. Das Gemisch wird innerhalb von 6 Stunden langsam auf 20°C abgekühlt. Die Suspension wird über einen Nutschfilter filtriert. Der Filterkuchen wird dann mit Aceton gewaschen und anschließend im Vakuum bei 40°C getrocknet. Es verbleiben 637g farblose Kristalle.

| | |
|---|---|
| HPLC (H₃PO₄): | logP = 0.50/1.00; |
| Massenspektrometrie: | 119.0 (Amin-M+H)⁺, 286.9 (Säure-M+H)⁺, chiral-HPLC 95.1 %ee; |
| ¹H-NMR [DMSO-D₆]: | 8.23 (br s, 3H), 7.70-7.71 (m, 1H), 7.45-7.46 (m, 1H), 7.35-7.36 (m, 2H), 7.22-7.23 (m, 2H), 4.35 (q, 1H), 3.07 (s, 3H), 2.31 (s, 3H), 1.47 (d, 3H). |

### Schritt 3: Herstellung von 2-Chlor-3 [(S)-methylsulfinyl]-4-(trifluoromethyl)benzoesäure

In einem gerührten Doppelmantelreaktor werden 4,9 l Eiswasser vorgelegt und 636g des Salzes aus Schritt 2 suspendiert. Dann werden insgesamt 0,55L einer konzentrierten Salzsäurelösung zugetropft und die Temperatur zwischen 0 °C und 5 °C gehalten. Die Suspension wird langsam auf Raumtemperatur erwärmt und über Nacht nachgerührt. Die Suspension wird dann über einer Nutschfilter filtriert Der Filterkuchen wird dann mit 3L destilliertem Wasser gewaschen und anschließend im Vakuum bei 50°C getrocknet. Es verbleiben 408,5g farblose Kristalle.

| | |
|---|---|
| HPLC (H₃PO₄): | logP = 1.00; |
| Massenspektrometrie: | 286.9 (M+H)⁺; chiral-HPLC 98.0 %ee; |
| ¹H-NMR [DMSO-D₆]: | 14.2 (br s, 1H) 7.96-7.99 (m, 2H), 3.14 (s, 3H). |

### Beispiel 2: Herstellung von 2-Chlor-N-(5-methyl-1,3,4-oxadiazol-2-yl)-3-[((S)-methylsulfinyl)]-4-(trifluormethyl)benzamid

28,6 g (0,1 mol) 2-Chlor-3 [(S)-methylsulfinyl]-4-(trifluoromethyl)benzosäure, 11 g (0,11 mol) 2-Amino-5-methyl-1,3,4-oxadiazol und 28,7 g (0,35 mmol) N-Methylimidazol werden in 200 ml Acetonitril gelöst und für 30 Minuten gerührt. Nach Abkühlen auf 5°C werden 18,9 g (0,16 mol) Thionylchlorid innerhalb von 60 Minuten so zugetropft, daß die Temperatur zwischen 5°C und 10°C bleibt. Danach wird noch 15 Stunden bei 20°C gerührt. Unter Vakuum wird das Lösungsmittel enfernt und der ölige Rückstand wird bei 40°C mit Wasser versetzt. Das Produkt fällt aus und wird nach Abfiltrieren mit kalter Salzsäure und Wasser gewaschen. Nach Trocknen erhält man 33,7 g (92 %) 2-Chlor-N-(5-methyl-1,3,4-oxadiazol-2-yl)-3-[((*S*)-methylsulfinyl)]-4-(trifluormethyl)benzamid mit einem Festpunkt von 220°C.

| | |
|---|---|
| Drehwert: | (-)-69 ° (MeOH). |

## Patentansprüche

1. Chirale 3-Sulfinylbenzoesäuren der in Formel (I-S) angegebenen absoluten Konfiguration 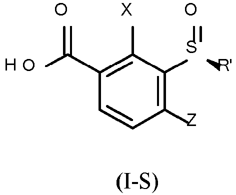 worin die Substituenten folgende Bedeutungen haben:
X bedeutet Cl oder Methyl,
R' bedeutet Methyl oder c-Pr,
Z bedeutet CF₃ oder CHF₂.

2. 3-Sulfinylbenzoesäuren gemäß Anspruch 1 mit einem Enantiomerenüberschuss (ee) von mindestens 94%.

3. 3-Sulfinylbenzoesäuren gemäß Anspruch 2 mit einem Enantiomerenüberschuss (ee) von mindestens 99%.

4. Verfahren zur Herstellung von 3-Sulfinylbenzoesäuren gemäß einem der Ansprüche 1 bis 3, **dadurch gekennzeichnet, daß**
a) racemische Verbindungen der Formel (I-rac) mit einem enantiomerenreinen Amin der allgemeinen Formel (II) umgesetzt werden,
b) eines der beiden auskristallisierten diastereomeren Salze (III-dr) oder (III-ds) abfiltriert, gereinigt und durch Zugabe von Wasser und Säure zur 3-Sulfinylbenzoesäure der Formel (I-R) oder (I-S) freigesetzt wird,
c) das andere diastereomere Salz aus der Mutterlauge gemäß Schritt a) durch Zugabe von Wasser und Säure zur 3-Sulfinylbenzoesäure der Formel (I-R) oder (I-S) freigesetzt wird, und
d) worin in Formel (II)
R¹ für Methyl, Ethyl, n-Propyl, iso-Propyl, n-Butyl, iso-Butyl steht, und
R² für Hydroxymethyl, Phenyl, 4-Methylphenyl steht:

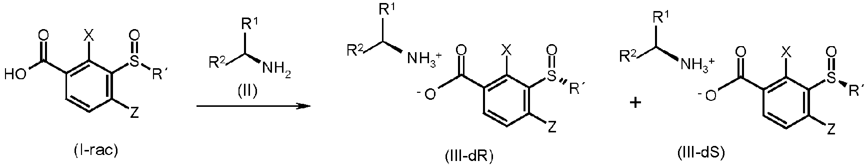

5. Verfahren zur Herstellung von N-(1,3,4-Oxadiazol-2-yl)phenylcarbonsäureamiden mit der in Formel (I*) angegebenen absoluten Konfiguration durch Umsetzung von 2-Amino-1,3,4-oxadiazolen der allgemeinen Formel (V) mit erfindungsgemäßen 3-Sulfinylbenzoesäuren der allgemeinen Formel (I-S), 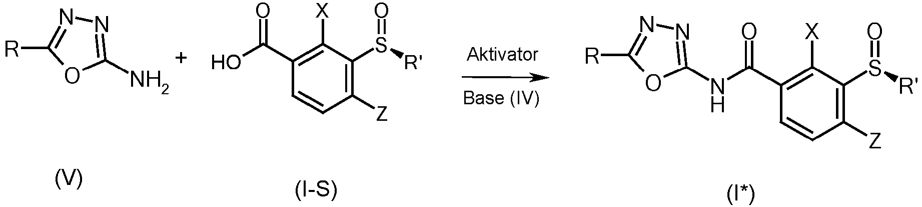 **dadurch gekennzeichnet, dass** es
a) in Gegenwart eines aktivierendes Reagenzes (Aktivator) aus der Gruppe bestehend aus Thionylchlorid, Phosgen, Diphosgen, Mesylchlorid, Tosylchlorid, POCl₃, PCl₅, Oxalylchlorid und C₁-C₈-alkyl-OC(O)Cl, und
b) in Gegenwart einer Base der allgemeinen Formel (IV) 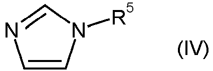 durchgeführt wird,
und
c) worin die Substituenten wie nachfolgend definiert sind:
R bedeutet Wasserstoff oder Methyl,
X bedeutet Cl oder Methyl,
R' bedeutet Methyl oder c-Pr,
Z bedeutet CF₃ oder CHF₂,
R⁵ bedeutet C₁-C₁₂-Alkyl oder Phenyl.

6. Verfahren nachAnspruch 5, worin die Verbindungen der Formeln (V) und (I-S) in einem Mol-Verhältnis von 0,8 bis 1,5 eingesetzt werden.

7. Verfahren nach Anspruch 5 oder 6, worin der Aktivator aus der Gruppe bestehend aus Thionylchlorid, Phosgen, Diphosgen, Mesylchlorid, Tosylchlorid, POCl₃, PCl₅, Oxalylchlorid und C₁-C₈-alkyl-OC(O)Cl ausgewählt ist und dieser Aktivator und die Verbindungen der Formel (I-S) in einem Mol-Verhältnis von 1 bis 2 eingesetzt werden.

8. Verfahren nach einem der Ansprüche 5 bis 7, worin der Aktivator aus der Gruppe bestehend aus Thionylchlorid, Phosgene oder Diphosgen ausgewählt ist.

## Claims

1. Chiral 3-sulfinylbenzoic acids of the absolute configuration indicated in formula (I-S) 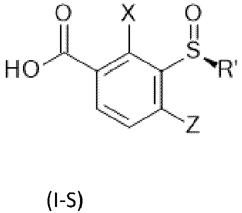 wherein the substituents have the following meanings:
X means Cl or methyl,
R´ means methyl or c-Pr,
Z means CF₃ or CHF₂.

2. 3-sulfinylbenzoic acids according to claim 1 with an enantiomeric excess (ee) of at least 94%.

3. 3-sulfinylbenzoic acids according to claim 2 with an enantiomeric excess (ee) of at least 99%.

4. Process for preparing 3-sulfinylbenzoic acids according to one of claims 1 to 3, **characterized in that**
a) reacting racemic compounds of formula (I-rac) with an enantiomerically pure amine of the general formula (II),
b) filtering off and purifying one of the two crystallized diastereomeric salts (III-dr) or (III-ds) and releasing it as the 3-sulfinylbenzoic acid of formula (I-R) or (I-S) by addition of water and acid,
c) releasing the other diastereomeric salt from the mother liquor according to step a) as the 3-sulfinylbenzoic acid of formula (I-R) or (I-S) by addition of water and acid, and
d) wherein in formula (II)
R¹ stands for methyl, ethyl, n-propyl, iso-propyl, n-butyl, iso-butyl, and
R² stands for hydroxymethyl, phenyl, 4-methylphenyl:

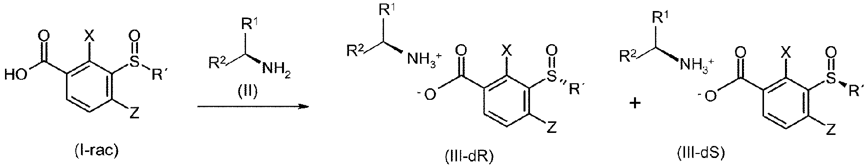

5. Process for preparing N-(1,3,4-oxadiazol-2-yl)phenylcarboxamides having the absolute configuration indicated in formula (I*) by reacting 2-amino-1,3,4-oxadiazoles of the general formula (V) with 3-sulfinylbenzoic acids according to the invention of the general formula (I-S), 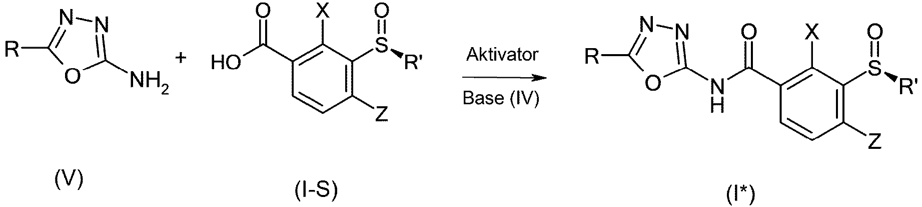 **characterized in that** it
a) is carried out in the presence of an activating reagent (activator) selected from the group consisting of thionyl chloride, phosgene, diphosgene, mesyl chloride, tosyl chloride, POCl₃, PCl₅, oxalyl chloride and C₁-C₈-alkyl-OC(O)Cl, and
b) in the presence of a base of the general formula (IV) 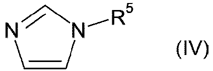 is carried out,
and
c) wherein the substituents are as defined below:
R means hydrogen or methyl,
X means Cl or methyl,
R´ means methyl or c-Pr,
Z means CF₃ or CHF₂,
R⁵ means C₁-C₁₂-alkyl or phenyl.

6. Process according to claim 5, wherein the compounds of formulas (V) and (I-S) are used in a molar ratio of 0.8 to 1.5.

7. Process according to claim 5 or 6, wherein the activator is selected from the group consisting of thionyl chloride, phosgene, diphosgene, mesyl chloride, tosyl chloride, POCl₃, PCl₅, oxalyl chloride and C₁-C₈-alkyl-OC(O)Cl, and this activator and the compounds of formula (I-S) are used in a molar ratio of 1 to 2.

8. Process according to one of claims 5 to 7, wherein the activator is selected from the group consisting of thionyl chloride, phosgene or diphosgene.

## Revendications

1. Acides 3-sulfinylbenzoïques chiraux de la configuration absolue indiquée dans la formule (I-S) 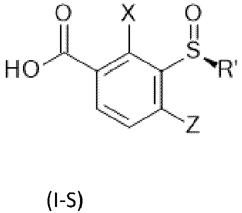 dans laquelle les substituants ont les significations suivantes :
X signifie Cl ou méthyle,
R´ signifie méthyle ou c-Pr,
Z signifie CF₃ ou CHF₂.

2. Acides 3-sulfinylbenzoïques selon la revendication 1 avec un excès énantiomérique (ee) d'au moins 94 %.

3. Acides 3-sulfinylbenzoïques selon la revendication 2 avec un excès énantiomérique (ee) d'au moins 99 %.

4. Procédé de préparation d'acides 3-sulfinylbenzoïques selon l'une des revendications 1 à 3, **caractérisé en ce que**
a) en faisant réagir des composés racémiques de la formule (I-rac) avec une amine énantiomériquement pure de formule générale (II),
b) en séparant par filtration l'un des deux sels diastéréoisomères cristallisés (III-dr) ou (III-ds), en le purifiant et en le libérant sous forme d'acide 3-sulfinylbenzoïque de formule (I-R) ou (I-S) par addition d'eau et d'acide,
c) en libérant l'autre sel diastéréoisomère de la liqueur mère selon l'étape a) sous forme d'acide 3-sulfinylbenzoïque de formule (I-R) ou (I-S) par addition d'eau et d'acide, et
d) dans laquelle, dans la formule (II)
R¹ représente méthyle, éthyle, n-propyle, iso-propyle, n-butyle, iso-butyle, et
R² représente hydroxyméthyle, phényle, 4-méthylphényle :

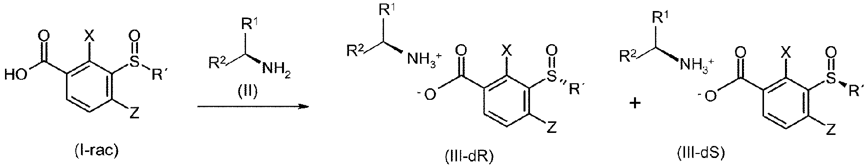

5. Procédé de préparation de N-(1,3,4-oxadiazol-2-yl)phénylcarboxamides ayant la configuration absolue indiquée dans la formule (I*) par faisant réagir des 2-amino-1,3,4-oxadiazoles de formule générale (V) avec des acides 3-sulfinylbenzoïques selon l'invention de formule générale (I-S), 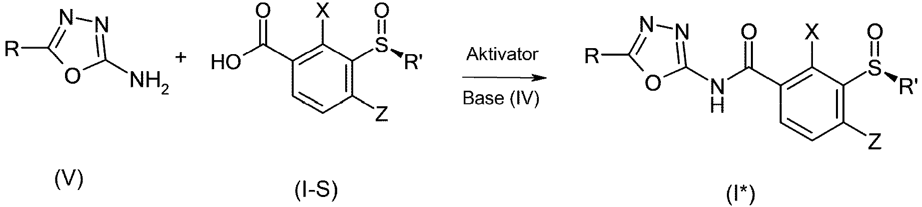 **caractérisé en ce qu'**il
a) est réalisé en présence d'un réactif activant (activateur) du groupe constitué de chlorure de thionyle, phosgène, diphosgène, chlorure de mésyle, chlorure de tosyle, POCl₃, PCl₅, chlorure d'oxalyle et C₁-C₈-alkyl-OC(O)Cl, et
b) en présence d'une base de formule générale (IV) 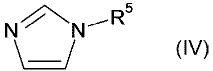 est réalisé,
et
c) dans laquelle les substituants sont définis comme suit :
R signifie hydrogène ou méthyle,
X signifie Cl ou méthyle,
R´ signifie méthyle ou c-Pr,
Z signifie CF₃ ou CHF₂,
R⁵ signifie C₁-C₁₂-alkyle ou phényle.

6. Procédé selon la revendication 5, dans lequel les composés des formules (V) et (I-S) sont utilisés dans un rapport molaire de 0,8 à 1,5.

7. Procédé selon la revendication 5 ou 6, dans lequel l'activateur est choisi dans le groupe constitué de chlorure de thionyle, phosgène, diphosgène, chlorure de mésyle, chlorure de tosyle, POCl₃, PCl₅, chlorure d'oxalyle et C₁-C₈-alkyl-OC(O)Cl et cet activateur ainsi que les composés de la formule (I-S) sont utilisés dans un rapport molaire de 1 à 2.

8. Procédé selon l'une des revendications 5 à 7, dans lequel l'activateur est choisi dans le groupe constitué de chlorure de thionyle, phosgène ou diphosgène.
